(19) Europäisches Patentamt
European Patent Office
Office européen des brevets

(11) Veröffentlichungsnummer: **0 217 206 B1**

(12) # EUROPÄISCHE PATENTSCHRIFT

(45) Veröffentlichungstag der Patentschrift: **03.11.93**

(51) Int. Cl.[5]: **A61K 31/42**, A61K 31/275

(21) Anmeldenummer: **86112687.8**

(22) Anmeldetag: **13.09.86**

Teilanmeldung 93106367.1 eingereicht am 13/09/86.

(54) **Arzneimittel gegen chronische Graft-versus-Host-Krankheiten sowie gegen Autoimmunerkrankungen, insbesondere systemischen Lupus erythematodes.**

(30) Priorität: **27.09.85 DE 3534440**

(43) Veröffentlichungstag der Anmeldung:
**08.04.87 Patentblatt 87/15**

(45) Bekanntmachung des Hinweises auf die Patenterteilung:
**03.11.93 Patentblatt 93/44**

(84) Benannte Vertragsstaaten:
**AT BE CH DE FR GB IT LI LU NL SE**

(56) Entgegenhaltungen:
**EP-A- 0 013 376**
**EP-A- 0 257 882**

**EUROPEAN JOURNAL OF IMMUNOLOGY, Band 12, 1982, Seiten 152-159, Verlag Chemie GmbH, Weinheim, DE; E. GLEICHMANN et al.: "A systemic lupus erythematosus (SLE)-like disease in mice induced by abnormal T-B cell cooperation. Preferential formation of autoantibodies characteristic of SLE"**

(73) Patentinhaber: **HOECHST AKTIENGESELLSCHAFT**

**D-65926 Frankfurt(DE)**

(72) Erfinder: **Bartlett, Robert R., Dr.**
**Sandbergstrasse 20**
**D-6100 Darmstadt(DE)**
Erfinder: **Schleyerbach, Rudolf, Dr.**
**Finkenweg 10**
**D-6238 Hofheim am Taunus(DE)**
Erfinder: **Kämmerer, Friedrich-Johannes, Dr.**
**Am Gänsborn 3a**
**D-6203 Hochheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

ANNUAL REPORTS IN MEDICINAL CHEMISTRY, Band 18, 1983, Seiten 171-179; V.J. STECHER et al.: "Disease modifying antirheumatic drugs"

INTERNATIONAL JOURNAL OF IMMUNOPHARMACOLOGY, Band 7, Nr. 1, Januar 1985, Seiten 7-18, International Society for Immunopharmacology; R.R. BARTLETT et al.: "Immunopharmacological profile of a novel isoxazol derivative, HWA 486, with potential antirheumatic activity. I. Disease modifying action on adjuvant arthritis of the rat"

INTERNATIONAL JOURNAL OF IMMUNOPHARMACOLOGY, Band 8, Nr. 2, April 1986, Seiten 199-204, International Society for Immunopharmacology; R.R. BARTLETT: "Immunopharmacological profile of HWA 486, a novel isoxazol derivative. II. in vivo immunodulating effects differ from those of cyclophosphamide, prednisolone, or cyclosporin A"

AGENT AND ACTIONS, Band 21, Nr. 3/4, 1987, Seiten 284-286, Birkhäuser Verlag, Basel, CH; S. POPOVIC et al.: "The use of the murine chronic graft Vs host (CGVH) disease, a model for systemic lupus erythematosus (SLE), for drugs discovery"

Oxford Textbook of Medicine, 2. Auflage, Band 2, S. 16.20-16.28 und S. 21.211-21.216

## Beschreibung

Aus der Europäischen Patentschrift 13 376 ist 5-Methylisoxazol-4-carbonsäure-(4-trifluormethyl)-anilid (Verbindung 1) als antiphlogistisch bekannt. Dort sind ebenfalls Verfahren zur Herstellung dieser Verbindung beschrieben.

Es wurde nun gefunden, daß diese Verbindung 1 und ihr Metabolit N-(4-Trifluormethylphenyl)-2-cyan-3-hydroxy-crotonsäureamid (Verbindung 2) [Stecher und Carlson, Ann. Report Med. Chem. 18, 171 - 179 (1983)] (Formeln s. Anspruch 1) solche immunmodulierenden Eigenschaften haben, daß sie sich als Arzneimittel gegen chronische Graft-versus-Host-Krankheiten (cGvH) sowie gegen Autoimmunerkrankungen, insbesondere systemischen Lupus erythematodes (SLE) eignen.

Gegenstand der Erfindung ist daher die Verwendung der beiden vorgenannten Verbindungen 1 und 2, wobei die Verbindung 2 als solche oder in Form eines physiologisch verträglichen Salzes verwendet werden kann, zur Herstellung von Arzneimitteln gegen chronische Graft-versus-Host-Krankheiten sowie gegen systemischen Lupus erythematodes. Ein weiterer Gegenstand der Erfindung ist die Verwendung der Verbindung 2 zur Herstellung von Arzneimitteln gegen Autoimmunerkrankungen. Geeignete Salze sind z. B. Alkali-, Erdalkali- und Ammoniumsalze einschließlich solcher von physiologisch verträglichen organischen Ammoniumbasen.

A) Chronische Graft-vs-Host (cGvh)-Krankheiten

Bei Transplantationen kommt es häufiger zur Abstoßung des Transplantats. Die Transplantat-Wirt-Beziehung beschränkt sich jedoch nicht allein auf die Abstoßung durch den Wirtsorganismus; in bestimmten Fällen kann eine vom Transplantat ausgehende, gegen das Wirtsgewebe gerichtete Immunreaktion eintreten. Man unterscheidet zwischen einer akuten und einer chronischen Reaktion. Die akute Graft-vs-Host-Reaktion ist durch Milzvergrößerung, Leberschwellung, Hypertrophie der Lymphknoten, hämolytische Anämie, niedrigen Immunglobulin- und Komplementspiegel sowie verminderte Immunreaktivität gekennzeichnet. Die akut verlaufende Reaktion endet fast immer tödlich.

Daneben gibt es die chronische Form des Krankheitsverlaufs. Sie führt zu Lymphadenopathie, Immunkomplexglomerulonephritis und zur Bildung von vielen Antikörpern. Diese Verlaufsform ist milder als die akute Form und führt nicht innerhalb kurzer Zeit zum Tode. Symptome, die durch diese cGvH-Reaktion ausgelöst werden, ähneln sehr stark denen des systemischen Lupus erythematodes.

B) Systemischer Lupus erythematodes (SLE)

Der systemische Lupus erythematodes ist eine nichtorganspezifische Autoimmunkrankheit. Diese Erkrankung befällt zahlreiche Organe und verläuft chronisch mit akuten Schüben. Äußere Erscheinungsbilder des SLE sind Hautveränderungen des Gesichts. Meist sind weitere Hautareale sowie die Schleimhaut befallen. Auch Nephritiden, Endokarditiden, hämolytische Anämien, Leukopenien und Beteiligung des Zentralnervensystems werden beobachtet.

Zahlreiche immunologische Phänomene wurden beim SLE beobachtet. Antikörper gegen bestimmte körpereigene Antigene werden gebildet. Diese Äntikörper, die sich bei SLE-Patienten nachweisen lassen, sind z.B. gegen die Basalmembran, der Haut, gegen Lymphozyten, Erythrozyten und nukleare Antigene gerichtet. In erster Linie bilden die gegen Doppelstrang-DNS (ds-DNS) gerichteten Antikörper mit diesen Komplexe, die sich zusammen mit Komplement auf kleinen Blutgefäßen ablagern und häufig zu Vasculitis führen. Diese Ablagerungen sind besonders gefährlich, wenn sie in den renalen Glomeruli vorkommen, da sie zu Glomerulonephritis und Nierenversagen führen. Die Häufigkeit der klinisch erfaßbaren Nierenbeteiligung wird in der Literatur mit 50 bis 80% angegeben.

Für das Überleben der Kranken mit systemischem Lupus erythematodes sind Glukokortikoide und andere immunsuppressive Medikamente, z.B. Cyclophosphamid (CPA) von entscheidender Bedeutung. Ein spezifisches Mittel zur Heilung gibt es bisher nicht. Die Therapie zielte bisher darauf ab, eine akute Exazerbation zu verhindern oder zu Überwinden und Rückfälle zu vermeiden. Zu diesem Zweck hat man die Patienten mit Glukokortikoiden und anderen Immunsuppressiva behandelt, die jedoch selbst gefährliche Nebenwirkungen haben.

Zur Erforschung des SLE gibt es verschiedene Tiermodelle. Bei einzelnen Mäusestämmen tritt ein spontaner SLE auf, wie bei den Neuseeland-Mäusen oder bei MRL/1-Mäusen, das sind ursprünglich aus den Jackson Laboratories, Maine, USA, stammende und In eigener Tierhaltung unter spezifisch pathogenfreien (SPF-)Verhältnissen weiter gezüchtete Tiere. Es ist aber auch möglich, durch einen experimentellen Ein-griff an nicht-autoimmunen Mäusen eine SLE-ähnliche Krankheit auszulösen.

Im folgenden beziehen sich die Mengenangaben mg/kg auf kg Körpergewicht; CMC bedeutet das Natriumsalz der Carb-oxymethylcellulose und o.P. "ohne Prüfsubstanz". In den Figuren 1 bis 5 ist die Verbindung 1 als Anilid 1 und die Verbindung 2 als Anilid 2 bezeichnet. Die Wirk-substanzen wurden oral im Gemisch mit CMC verabreicht. "N.K." bedeutet Negativ-Kontrolle.

C) Pharmakologische Prüfungen und Ergebnisse

1) Die chronische Graft-vs-Kost-Krankheit

Dieses Modell wurde von GLEICHMANN et al. in verschiedenen Veröffentlichungen beschrieben. Der SLE wird dadurch induziert, daß eine GvH-Reaktion durch eine abnormale T-B-Zellkooperation ausgelöst wird [GLEICHMANN et al., Euro.J.Immunol. 12; 152-159 (1982)]. Die cGvH-Krankheit wurde durch zwei Injektionen von Milz- und Thymuszellen ausgelöst. Die Mäuse, gemäß GLEICHMANN et al. (DBA/2 x C57B1/6)F1-Generation, erhielten jeweils 70 x $10^6$ DBA/2-Zellen am 1. Tag und 8. Tag, die in 0,2 ml Kulturmedium intravenös injiziert wurden. Die Behandlung der Tiere erfolgte erstmals am 17. Tag nach der ersten Injektion der Donorzellen. Es wurden drei unabhängige Versuche 1.1 bis 1.3 durchgeführt, wobei in allen drei Experimenten nur weibliche Tiere als Spender und als Empfänger benutzt wurden. Jedem Tier wurde 1 ml oral verabreicht, der jeweils die in den Versuchen 1.1 bis 1.3 angegebene Menge Wirkstoff und daneben CMC in einer Konzentration von 100 mg/l enthielt.

Versuche

1.1) Es wurden vom 17. Tag an einmal täglich verabreicht: CMC, o.P.,
8 mg/kg CPA bzw.
5 mg/kg Verbindung 1 bzw.
10 mg/kg Verbindung 1 bzw.
20 mg/kg Verbindung 1.
In der Negativkontrolle (Tiere ohne cGvH) befanden sich in jeder Gruppe 10 Tiere und in den übrigen Gruppen jeweils 18 Tiere.
1.2) Es wurden vom 17. Tag an verabreicht CMC, o.P. zweimal pro Woche,
28 mg/kg Verbindung 1 einmal täglich bzw.
14 mg/kg CPA zweimal pro Woche bzw.
28 mg/kg CPA zweimal pro Woche bzw.
50 mg/kg CPA zweimal pro Woche.
In jeder cGvH-Gruppe waren 20-21 Tiere, in der Negativkontrolle je 9 Tiere.
1.3) Es wurden vom 17. Tag an einmal täglich verabreicht CMC, o.P.,
1 mg/kg Indomethacin bzw.
2 mg/kg Prednisolon bzw.
20 mg/kg Verbindung 2 bzw.
30 mg/kg Verbindung 2.
In jeder Gruppe waren 10 - 11 Tiere.

Proteinurie

Während der Dauer des Versuchs (9-10 Wochen) wurde wöchentlich die Menge an Protein im Urin der Tiere festgestellt (Albu-sticks-Reagenzstäbchen, Ames Division, Miles Laboratories, Elkhard). Die Ergebnisse dieser Untersuchungen sind in den Figuren 1-3 dargestellt.

b) Hemmung des Graft-vs-Host-Index

Im Verlaufe einer cGvH-Krankheit wird die Milz infolge der Immunabwehr wesentlich vergrößert. Wenn man das Gewicht der Milz in Relation zum Körpergewicht des erkrankten Tieres setzt und dieses Verhältnis mit der entsprechenden Relation beim gesunden Tier vergleicht, erhält man den Graft-vs-Host-Index:

$$\text{GvH-Index} = \frac{\text{Milzgewicht X / Körpergewicht X}}{\text{Milzgewicht g / Körpergewicht g}}$$

wobei X = untersuchte (kranke) Tiere und g = gesunde Tiere.

Der GvH-Index ermöglicht die Feststellung der Krankheitsstärke: je größer der Index, desto stärker die Krankheit. Die Ergebnisse sind in Tabelle 2 wiedergegeben.

TABELLE 1

| Versuch | Substanz | GvH-Index | % Änderung |
|---|---|---|---|
| 1.1 | CMC, o.P. | 2,06 | 0 |
| 1.1 | 8 mg/kg CPA je Tag | 1,03 | -50,0 |
| 1.1 | 5 mg/kg Verbindung 1 je Tag | 2,00 | - 2,9 |
| 1.1 | 10 mg/kg Verbindung 1 je Tag | 1,97 | - 4,4 |
| 1.1 | 20 mg/kg Verbindung 1 je Tag | 1,67 | -19,9 |
| 1.2 | CMC, o.P. | 2,66 | 0 |
| 1.2 | 28 mg/kg Verbindung 1 je Tag | 1,37 | -48,5 |
| 1.2 | 14 mg/kg CPA 2x wöchentlich | 1,39 | -47,7 |
| 1.2 | 28 mg/kg CPA 2x wöchentlich | 1,29 | -51,5 |
| 1.2 | 50 mg/kg CPA 2x wöchentlich | 1,21 | -54,5 |
| 1.3 | CMC, o.P. | 2,99 | 0 |
| 1.3 | 1 mg/kg Indomethacin je Tag | 3,44 | 115 |
| 1.3 | 2 mg/kg Prednisolon je Tag | 1,27 | - 58 |
| 1.3 | 20 mg/kg Verbindung 2 je Tag | 1,99 | - 33 |
| 1.3 | 30 mg/kg Verbindung 2 je Tag | 1,27 | - 56 |

2) MRL-lpr/lpr-Mäuse (MRL/1) als Modell für SLE

Bei diesen Tieren tritt eine spontane SLE-Krankheit auf. Die MRL/1-Mäuse besitzen Antikörper gegen nukleare Bestandteile, Hypergammaglobuline und zirkulierende Immunkomplexe. Die Glomerulonephritis ist normalerweise die Todesursache.

Versuch 2.1

Untersucht wurden die Effekte der Verbindung 1 auf die Entwicklung der SLE-Krankheit in männlichen und weiblichen MRL/1-Mäusen. Nachdem die Tiere 9 Wochen alt waren, wurden sie in Gruppen aufgeteilt (n = 20) und die Behandlung mit der Substanz begonnen. Jedem Tier wurde 1 ml oral verabreicht, der jeweils die im Versuch 2.1 angegebene Menge Wirkstoff und daneben CMC in einer Konzentration von 100 mg/l enthielt.

Unbehandelte MRL/1-Mäuse (Positiv-Kontrolle)
MRL/1 Mäuse 5 mg/kg Verbindung 1 je Tag
MRL/1 Mäuse 20 mg/kg Verbindung 1 je Tag
MRL/1 Mäuse 28 mg/kg Verbindung 1 je Tag
MRL/1 Mäuse 35 mg/kg Verbindung 1 je Tag
unbehandelte NMRI-Mäuse (Negativ-Kontrolle)

a) Proteinurie

Die Proteinausscheidung im Urin wurde, wie unter 1a) beschrieben, gemessen. Die Ergebnisse von weiblichen Tieren sind in Figur 4 dargestellt; die Ergebnisse bei männlichen Tieren waren ähnlich.

b) Titer von Antikörpern gegen Doppelstrang-DNS (ds-DNS)

SLE ist durch Antikörper gegen nukleare Bestandteile gekennzeichnet. Die anti-ds-DNS-Antikörper der IgG-Klasse sind SLE-spezifisch und werden für die Diagnostik verwendet. Nachdem die Tiere 35 Wochen alt waren, wurden sie entblutet und die Antikörper-Titer des Serums bestimmt - unter Anwendung einer ELISA-Methode [Enzyme Linked Immunosorbent Assay; Kávai et al., J. Immunol. Meth. 48, 169-175 (1982); Pisetsky et al., J. Immunol. Methods 74, 217-227 (1984)]. Die Ergebnisse sind in Tabelle 3 zusammengefaßt.

TABELLE 3

| Substanz | Titer (Mittelwert) | Bereich |
|---|---|---|
| unbehandelt | 8145 | 3200-12800 |
| 5 mg/kg Verbindung 1 je Tag | 8533 | 3200-12800 |
| 20 mg/kg Verbindung 1 je Tag | 3844 | 1600- 6400 |
| 28 mg/kg Verbindung 1 je Tag | 1000 | 400- 3200 |
| 35 mg/kg Verbindung 1 je Tag | 470 | 50- 2400 |
| unbehandelte +NMRI-Mäuse | unter 100 | --- |

+) NMRI = Naval Medical Research Institute

c) Proliferierbarkeit von T-Lymphozyten

Obwohl eine massive Proliferation von T-Lymphozyten-Unterklassen bei MRL/1-Mäusen vorhanden ist, ist die Proliferation durch Mitogene vermindert. Es wird angenommen, daß diese abnormale T-Zellenfunktion eine fundamentale ätiologische Rolle bei der autoimmunen Krankheit der MRL/1-Mäuse spielt. Eine therapeutische Wiederherstellung dieser verminderten T-Zellenfunktion wäre günstig.

Die Milz wurde steril entnommen und wie von BARTLETT und SCHLEYERBACH [Int.J. Immunopharmacol. 7, 7-18 (1985)] beschrieben, behandelt. Die Ergebnisse in Figur 5 zeigen, daß die Verbindung 1 eine dosisabhängige Verbesserung der durch Phytohämagglutinin (PHA) und Concanavalin A (ConA) stimulierten T-Lymphozyten hervorruft. Die PHA-induzierte Proliferation ist sogar von gleicher Stärke wie die Lymphozyten von nicht-autoimmunen NMRI-Mäusen.

Die Ergebnisse der vorstehenden Untersuchungen zeigen, daß die Verbindungen 1 und 2 die Entstehung von cGvH- und SLE-Krankheiten in Mäusen hemmen.

Durch diese Substanzen wird

1) die Entstehung einer Glomerulonephritis bei beiden Krankheiten verhindert; dies konnte durch die verminderte Proteinausscheidung im Urin und durch histologische Untersuchungen an Nieren gezeigt werden;
2) der Anti-ds-DNS-Antikörpertiter herabgesetzt;
3) der GvH-Index vermindert;
4) die verminderte T-Lymphozyten-Proliferation dosisabhängig verbessert.

Die Verbindungen 1 und 2 zeigen Vorteile gegenüber Immunsuppressiva wie Cyclophosphamid oder Glukokortikoiden, da sie keine allgemeine Suppression des Immunsystems verursachen und sogar die Wiederherstellung der verminderten T-Zellenfunktion ermöglichen. Um so überraschender ist die Tatsache, daß sie gegen die chronischen GvH-Krankheiten und die SLE gut wirksam sind.

Die Verbindungen 1 und 2 können entweder allein, gegebenenfalls in Form von Mikrokapseln, oder vermischt mit üblichen physiologisch verträglichen Trägerstoffen, Verdünnungsmitteln und/oder Konstituentien verabreicht werden. Die Mittel können oral, rektal, intravenös oder parenteral verabreicht werden, wobei die orale oder rektale Anwendung bevorzugt ist. Geeignete feste oder flüssige galenische Zubereitungsformen sind beispielsweise Granulate, Pulver, Tabletten, Dragees, Kapseln, Zäpfchen, Sirupe, Emulsionen, Suspensionen, Aerosole, Tropfen oder injizierbare Lösungen in Ampullenform, wobei auch die Trockenampulle als eine spezielle Zubereitungsform eingeschlossen ist, sowie Präparate mit protrahierter Wirkstoff-Freigabe, bei deren Herstellung gewöhnlich Hilfsmittel, wie Trägerstoffe, Spreng-, Binde, Überzugs-, Quellungs-, Gleit- oder Schmiermittel, Geschmacksstoffe, Süßungsmittel, Puffersubstanzen, Antioxidantien und/oder Lösungsvermittler, Verwendung finden. Häufig verwendete Hilfsstoffe sind z.B. Magnesium- oder Calciumcarbonat, Calciumphosphate, Titandioxid, Mannit, Laktose und andere Zucker, Talkum, Milcheiweiß, Gelatine, Stärke, Vitamine, Cellulose und ihre Derivate, tierische und pflanzliche Öle, Polyäthylenglykole und physiologisch unbedenkliche Lösungsmittel, wie steriles Wasser, Alkohole, Glycerin und andere mehrwertige Alkohole.

Vorzugsweise werden die pharmazeutischen Präparate in Dosierungseinheiten hergestellt und verabreicht, wobei jede Einheit als aktiven Bestandteil eine bestimmte Dosis der Verbindung 1 und/oder 2 enthält. Bei festen Dosierungseinheiten, wie Tabletten, Kapseln und Suppositorien, kann diese Dosis von 10 bis 200 mg, bevorzugt jedoch 50 bis 100 mg, bei Injektionslösungen in Ampullenform (intravenös), insbesondere

auf Basis der Verbindung 2 bzw. eines Salzes davon, 1 bis 30 mg, vorzugsweise 5 bis 10 mg und bei rektaler Verabreichung 50 bis 300 mg, vorzugsweise 100 bis 200 mg betragen.

Für die Behandlung eines erwachsenen Patienten sind am Menschen Tagesdosen von 50 bis 200 mg Wirkstoff bei oraler Verabreichung, von 10 bis 30 mg bei intravenöser Applikation und von 100 bis 300 mg bei rektaler Applikation indiziert. Unter Umständen können jedoch auch höhere oder niedrigere Tagesdosen empfehlenswert sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Eine weitere Anwendung der Verbindungen besteht in der Kombination mit anderen geeigneten Wirkstoffen, beispielsweise Antiuricopathika, Thrombocytenaggregationshemmern, Analgetika und steroidalen oder nichtsteroidalen Antiphlogistika.

## Patentansprüche

1. Verwendung der Verbindung 1 und/oder 2 der Formeln

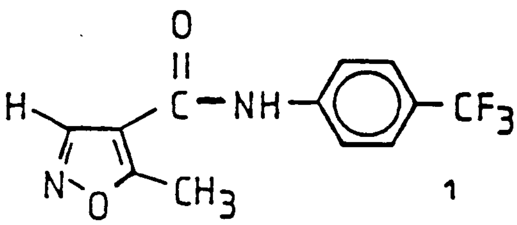

1

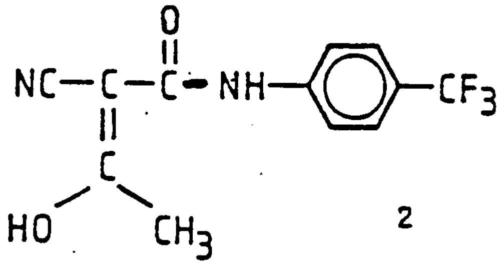

2

wobei die Verbindung 2 als solche oder in Form eines physiologisch verträglichen Salzes vorliegt, zur Herstellung von Arzneimitteln gegenchronische Graft-versus-Host-Krankheiten sowie gegen systemischen Lupus erythematodes.

2. Verwendung nach Anspruch 1, dadurch gekennzeichnet, daß das Arzneimittel in oral verabreichbarer Form vorliegt.

3. Verwendung nach Anspruch 1, dadurch gekennzeichnet, daß das Arzneimittel in rektal verabreichbarer Form vorliegt.

4. Verwendung nach Anspruch 1, dadurch gekennzeichnet, daß das Arzneimittel mit einem Gehalt an der Verbindung der Formel 2 als solcher oder in der Form eines Salzes in Form einer Injektionslösung vorliegt.

5. Dosierungseinheit eines Arzneimittels, enthaltend eine wirksame Menge der Verbindung 2, wobei die Verbindung 2 als solche oder in der Form eines Salzes vorliegt.

6. Dosierungseinheit nach Anspruch 5, dadurch gekennzeichnet, daß sie für die orale Anwendung bestimmt ist und 10 bis 200 mg, vorzugsweise 50 bis 100 mg der Verbindung 2 enthält, wobei die Verbindung 2 als solche oder in der Form eines Salzes vorliegt.

7. Dosierungseinheit nach Anspruch 5, dadurch gekennzeichnet, daß sie für die intravenöse Verabreichung gemäß Anspruch 4 bestimmt ist und die Verbindung 2 als solche oder in Form eines Salzes in einer Menge von 1 bis 30 mg, vorzugsweise 5 bis 10 mg enthält.

8. Dosierungseinheit nach Anspruch 5, dadurch gekennzeichnet, daß sie für die rektale Verabreichung bestimmt ist und die Verbindung 2 in einer Menge von 50 bis 300 mg,vorzugsweise 100 bis 200 mg enthält, wobei die Verbindung 2 als solche oder in der Form eines Salzes vorliegt.

9. Verwendung der Verbindung 2 nach Anspruch 1, wobei die Verbindung 2 als solche oder in Form eines physiologisch verträglichen Salzes vorliegt, zur Herstellung von Arzneimitteln gegen Autoimmunerkrankungen.

## Claims

1. Use of compound 1 and/or 2 of the formulae

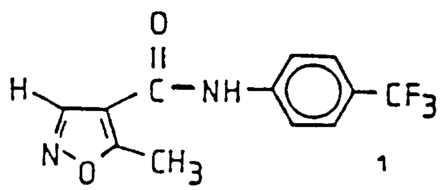
1

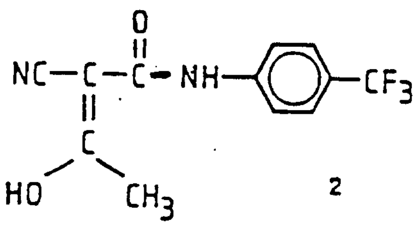
2

compound 2 being present as such or in the form of a physiologically tolerated salt, for the preparation of medicaments to combat chronic graft-versus-host diseases and to combat systemic lupus erythematosus.

2. The use as claimed in claim 1, wherein the medicament is present in a form suitable for oral administration.

3. The use as claimed in claim 1, wherein the medicaments are present in a form suitable for rectal administration.

4. The use as claimed in claim 1, wherein the medicament is present in the form of a solution for injection, with a content of the compound of the formula 2 as such or in the form of a salt.

5. A dosage unit of a medicament, which contains an effective amount, of compound 2, compound 2 being present as such or in the form of a salt.

6. The dosage unit as claimed in claim 5, which is intended for oral administration and contains 10 to 200 mg, preferably 50 to 100 mg, of compound 2 being present as such or in the form of a salt.

7. The dosage unit as claimed in claim 5, which is intended for intravenous administration as claimed in claim 4 and contains compound 2 as such or in the form of a salt in an amount of 1 to 30 mg, preferably 5 to 10 mg.

8. The dosage unit as claimed in claim 5, which is intended for rectal administration and contains compound 2 in an amount of 50 to 300 mg, preferably 100 to 200 mg, compound 2 being present as such or in the form of a salt.

9. The use of compound 2 as claimed in claim 1, compound 2 being present as such or in the form of a physiologically tolerated salt, for the preparation of medicaments to combat autoimmune diseases.

## Revendications

1. Utilisation du composé 1 et/ou 2 de formules

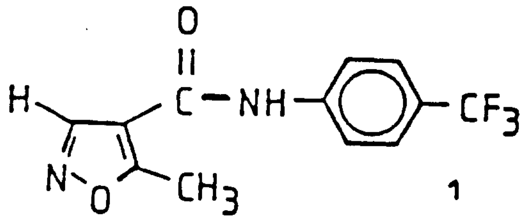

1

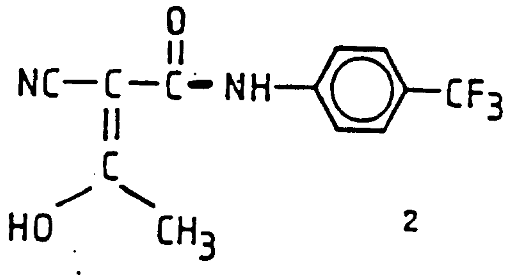

2

le composé 2 se trouvant tel quel ou sous forme d'un sel physiologiquement acceptable, pour la fabrication de médicaments contre des maladies chroniques de la réaction du greffon contre l'hôte, ainsi que contre le lupus érythémateux disséminé.

2. Utilisation selon la revendication 1, caractérisée en ce que le médicament se trouve sous une forme apte à l'administration orale.

3. Utilisation selon la revendication 1, caractérisée en ce que le médicament se trouve sous une forme apte à l'administration rectale.

4. Utilisation selon la revendication 1, caractérisée en ce que le médicament, ayant une teneur en le composé de formule 2, tel quel ou sous la forme d'un sel, se trouve sous forme d'une solution injectable.

5. Dose unitaire d'un médicament contenant une quantité efficace du composé 2, le compose 2 se trouvant tel quel ou sous la forme d'un sel.

6. Pose unitaire selon la revendication 5, caractérisée en ce qu'elle est destinée à l'administration orale et contient de 10 à 200 mg, de préférence de 50 à 100 mg du composé 2, le composé 2 se trouvant tel quel ou sous la forme d'un sel.

7. Pose unitaire selon la revendication 5, caractérisée en ce qu'elle est destinée à l'administration intraveineuse selon la revendication 4, et contient le composé 2 tel quel ou sous forme d'un sel, en une quantité de 1 à 30 mg, de préférence de 5 à 10 mg.

8. Pose unitaire selon la revendication 5, caractérisée en ce qu'elle est destinée à l'administration rectale et contient le composé 2 en une quantité de 50 à 3OO mg, de préférence de 100 à 200 mg, le composé 2 se trouvant tel quel ou sous la forme d'un sel.

9. Utilisation du composé 2 selon la revendication 1, le composé 2 se trouvant tel quel ou sous forme d'un sel physiologiquement acceptable, pour la fabrication de médicaments contre des maladies auto-immunes.

FIG. 1

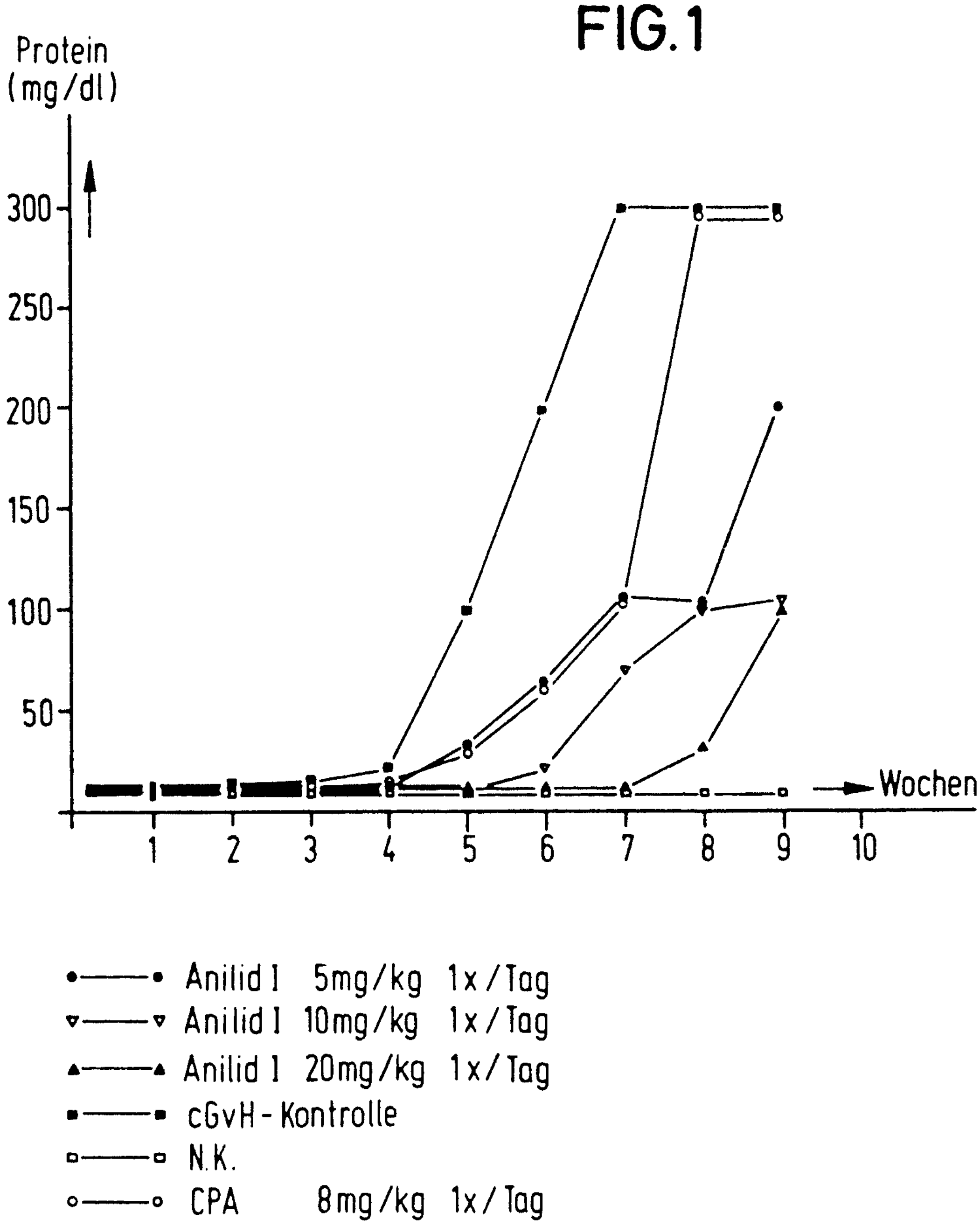
Protein
(mg/dl)
300
250
200
150
100
50
Wochen
1
2
3
4
5
6
7
8
9
10
Anilid I 5mg/kg 1x/Tag
Anilid I 10mg/kg 1x/Tag
Anilid I 20mg/kg 1x/Tag
cGvH-Kontrolle
N.K.
CPA 8mg/kg 1x/Tag

FIG. 2

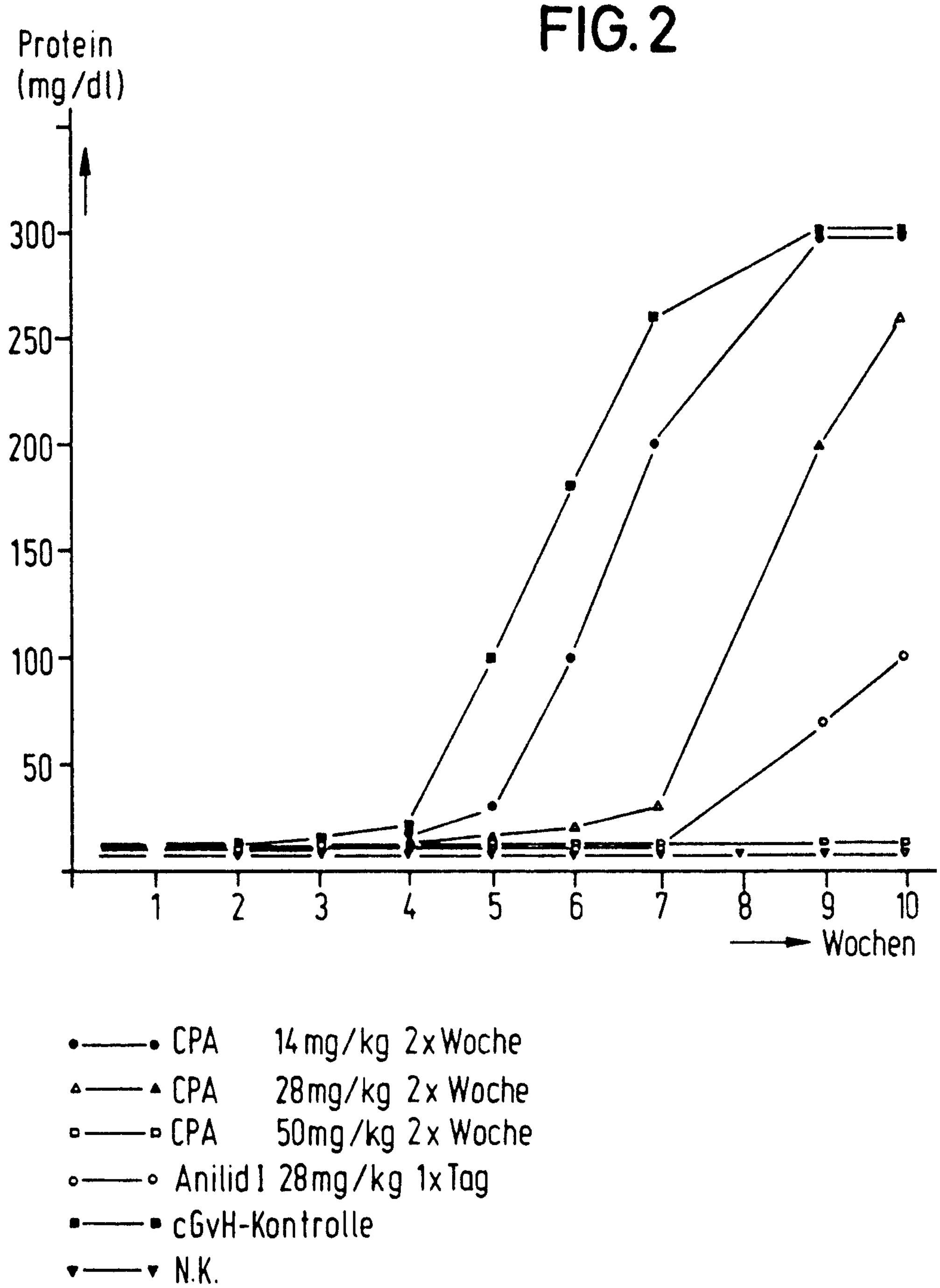
Protein
(mg/dl)
300
250
200
150
100
50
1
2
3
4
5
6
7
8
9
10
Wochen
CPA 14 mg/kg 2 x Woche
CPA 28 mg/kg 2 x Woche
CPA 50 mg/kg 2 x Woche
Anilid I 28 mg/kg 1 x Tag
cGvH-Kontrolle
N.K.

FIG. 3

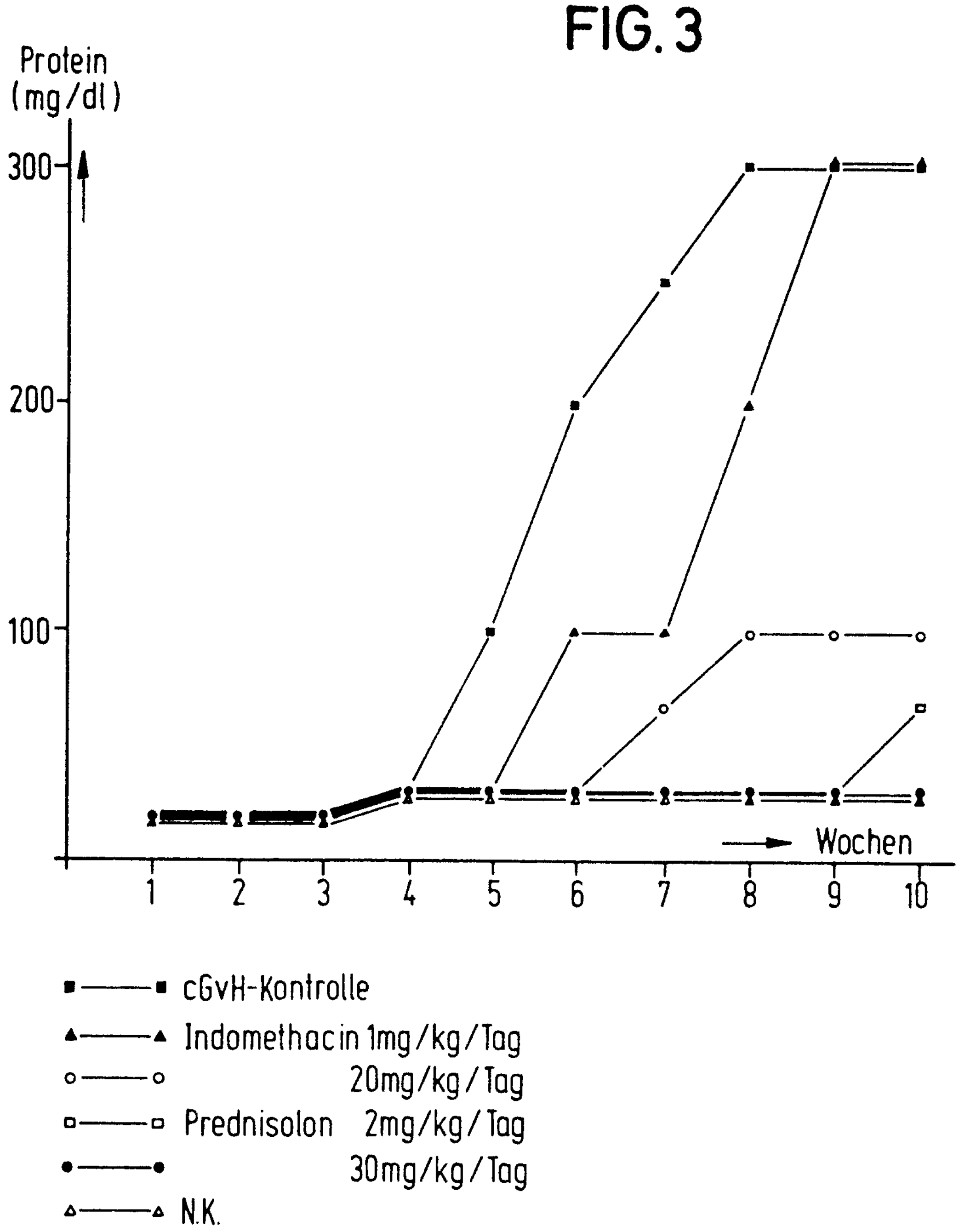
Protein
(mg/dl)
300
200
100
Wochen
1
2
3
4
5
6
7
8
9
10
cGvH-Kontrolle
Indomethacin 1mg/kg/Tag
20mg/kg/Tag
Prednisolon 2mg/kg/Tag
30mg/kg/Tag
N.K.

FIG.4

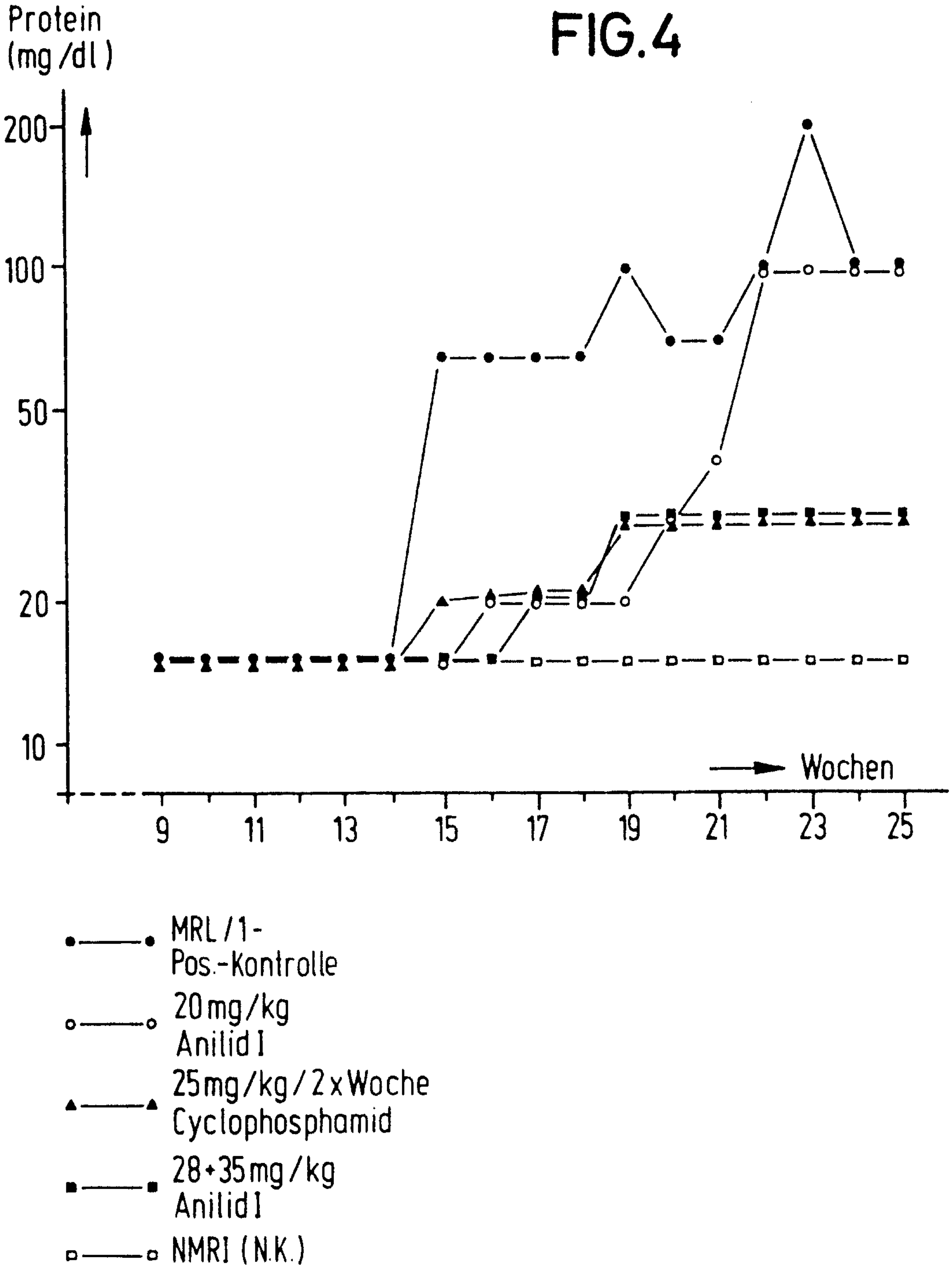
Protein
(mg/dl)
200
100
50
20
10
Wochen
9
11
13
15
17
19
21
23
25
MRL/1-
Pos.-Kontrolle
20mg/kg
Anilid I
25mg/kg/2xWoche
Cyclophosphamid
28+35mg/kg
Anilid I
NMRI (N.K.)

FIG.5

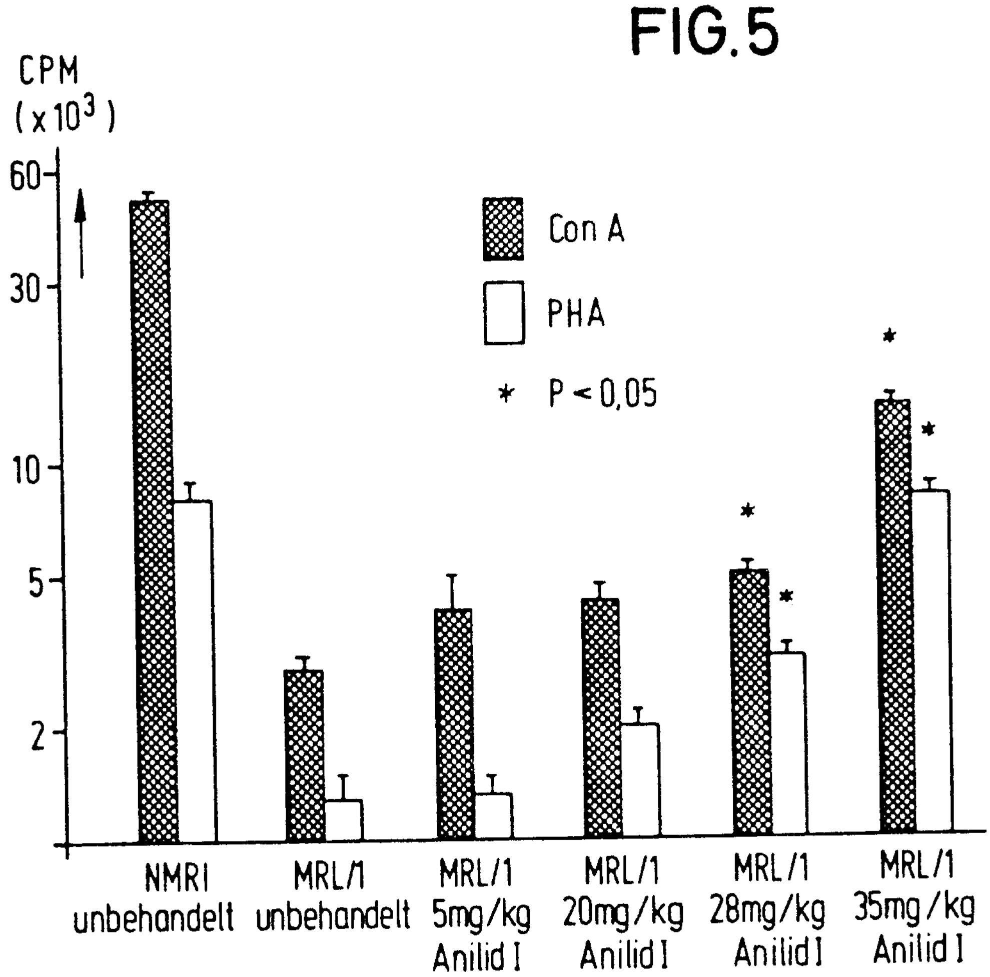
CPM
(x10³)
60
30
10
5
2
Con A
PHA
* P < 0,05
NMRI unbehandelt
MRL/1 unbehandelt
MRL/1 5mg/kg Anilid I
MRL/1 20mg/kg Anilid I
MRL/1 28mg/kg Anilid I
MRL/1 35mg/kg Anilid I